# EUROPEAN PATENT SPECIFICATION

(11) **EP 1 722 749 B1**
(45) Date of publication and mention of the grant of the patent: **25.06.2008**
(21) Application number: 05708342.0
(22) Date of filing: 15.02.2005
(51) Int. Cl.: A61K 8/02, A61K 8/22, A61Q 19/00

(54) **SKINCARE COMPOSITIONS CONTAINING SALICYLIC ACID**
HAUTPFLEGEMITTEL DIE SALICYLSÄURE ENTHALTEN
COMPOSITION DE SOIN DE LA PEAU COMPRENANT DE L' ACIDE SALICYLIQUE

(30) Priority: 19.02.2004 GB 0403702
(43) Date of publication of application: 22.11.2006
(73) Proprietor: Reckitt & Colman (Overseas) Limited, Slough Berkshire SL1 3UH (GB)
(72) Inventor: EVISON, Jane, Hull HU8 7DS (GB)
(74) Representative: Cawdell, Karen Teresa
(86) International application number: PCT/GB2005/000524
(87) International publication number: WO 2005/079746

(56) References cited:
- WO-A-01/28338
- WO-A-95/16454
- WO-A-03/022236
- WO-A-03/022237
- WO-A-20/05025486
- US-A- 5 693 318
- US-A- 5 736 582
- US-A- 5 786 346
- US-A1- 2002 031 556
- US-A1- 2002 172 719
- US-B1- 6 428 772
- PATENT ABSTRACTS OF JAPAN vol. 008, no. 034 (C-210), 15 February 1984 (1984-02-15) & JP 58 198421 A (REIKO KOSAKA), 18 November 1983 (1983-11-18)

## Description

This invention relates to skincare compositions, and in particular to compositions in the form of hydroalcoholic gels containing salicylic acid, especially gels that may have an astringent or toning effect on the skin, and to uses that involve the application of such compositions.

Acne vulgaris (acne) is a chronic inflammatory condition of the pilosebaceous units of the skin, which is particularly prevalent in adolescents. The condition generally causes the formation, on the skin, of comedones, red papules, pustules and sometimes cysts. This is unsightly and furthermore, if untreated, acne can lead to scarring of the skin. The major causes of acne are thought to be an increase in sebum production, an increased presence of *propionibacterium acne* (*P. acne*), blockage of the pilosebaceus duct and the production of inflammation.

Salicylic acid is known to be effective in the treatment of acne. It is a topical keratolytic agent that works by dissolving the intercellular cement that holds epithelial cells together. Salicylic acid is used in a variety of over-the-counter acne remedies.

Skincare compositions are typically formulated at approximately the pH of the skin, namely at or around pH 5.5. This optimises compatibility with the skin and aids transport of active molecules onto and through the skin. However, when foi-mulating salicylic acid, it is desired to provide a gel which has a more acidic pH, for example a pH of 4.5 or less. Salicylic acid is most effective when applied topically in an acidic environment. A further formulation issue is that, due to its poor solubility characteristics, salicylic acid is difficult to solubilise to provide stable compositions.

Astringent compositions are used to "tone" and moisturise the skin. Astringent compositions typically comprise a solvent system including a high proportion of a relatively volatile solvent, most commonly ethanol or isopropyl alcohol. Such compositions may comprise as much as 40% or more alcohol and are commonly of low viscosity.

The low viscosity of the composition means that it spreads easily and well when applied to the skin, but has the disadvantage that the composition may be difficult to apply. As a result, a large proportion of the product may be lost when the composition is transferred from the packaging to the user's hands, and then from the hands to the intended site of application, most commonly the face and surrounding areas. This leads to wastage and may consequently result in dissatisfaction on the part of the consumer. If, alternatively, the composition is applied using an absorbent pad or the like, then large proportions of the composition may be absorbed directly into the pad and again be wasted.

Wastage of the composition may be reduced by increasing the viscosity, eg by formulation of the composition as a gel. However, this has the disadvantage that the composition may be more difficult to apply. In addition, many gelling agents do not form effective gels at low pH to form a cosmetically acceptable hydroalcoholic gel which solubilises salicylic acid adequately and is also stable on storage. Furthermore, gelling agents useful at low pH may not function adequately in hydroalcoholic systems as the alcohol changes the solvation characteristics of the polymer in the aqueous system. In addition, on application to the skin, the alcohol evaporates off as the gel is rubbed into the skin. Again, it is important to ensure that this does not have a deleterious effect on the cosmetic acceptability of the product at the point of use.

For the above reasons, it would be desirable to develop an improved skincare composition that is sufficiently viscous to be relatively easy to dispense and handle prior to application, yet which spreads easily upon the intended site of application.

It is known to use taurate copolymers as thickening agents in aqueous systems. For example, WO 03022236 discloses a cosmetic composition comprising 0.01 to 20% C₁₋₂₅ alpha- or beta-hydroxycarboxylic acid at least partially present as a salt, 0.01 to 10% of a taurate copolymer and a cosmetically acceptable carrier, wherein the composition has a pH of less than 6. In addition, WO 03022237 discloses a cosmetic composition comprising 0.001 to 5% of a polysaccharide gum, 0.001 to 10% of a taurate copolymer and a cosmetically acceptable carrier, wherein the composition has a pH of less than 7. US 6620420 discloses a gel-cream of the oil-in-water type comprising up to 90% water, up to 20% lipid phase, up to 5% emulsifiers and up to 5% one or more ammonium acroyldimethyltaurate/vinylpyrrolidone copolymers. However, these disclosures relate to systems to provide thickened cosmetic compositions effective at low pH which are of sufficiently aesthetically pleasing viscosity and skinfeel. None of the above documents suggest the use of these systems in hydroalcoholic gels suitable for solubilising salicylic acid.

There has now been developed a skincare formulation that substantially satisfies the above-described requirements and/or which overcomes or substantially mitigates the above-mentioned and/or other disadvantages associated with the prior art.

Accordingly, there is provided a cosmetically acceptable skincare composition in the form of a hydroalcoholic gel dispersion, the composition comprising salicylic acid or a salt thereof and a gelling agent in the form of a copolymer of acryloyl dimethyl tauric acid or a salt thereof.

WO 0128338 discloses topical compositions comprising a benzoic acid analogue, a metal salt and a carrier wherein the composition has a pH from 1-7. The combination of the benzoic acid analogue and metal salt are said to provide a synergistic immediate and residual anti-viral and antibacterial efficacy. One of the example compositions disclosed therein comprises a hydroalcoholic gel comprising a combination of acrylamidomethylpropane sulphonic acid (available under the trade name Aritoflex AVC) and xanthan gum as thickening agents. However, it has been found that the gel provided in accordance with that disclosure has an intense colour and an unpleasant odour. In addition, due to the presence of the metal salt, the viscosity of the gel increases on storage. Accordingly, a hydroalcoholic gel containing salicylic acid, acrylamidomethylpropane sulphonic acid (available under the trade name Aristoflex AVC), xanthan gum and the polyvalent iron trichloride is not cosmetically acceptable.

WO 2005/025486 (published 24 March 2005) discloses skincare compositions comprising hydrogen peroxide and salicylic acid. As an optional ingredient there may be included a copolymer of acryloyl dimethyl tauric acid or a salt thereof.

According to the invention, there is provided a cosmetically acceptable skincare composition in the form of a hydroalcoholic gel dispersion, the composition comprising salicylic acid or a salt thereof and a gelling agent in the form of a copolymer of acryloyl dimethyl tauric acid or a salt thereof, provided that if the composition contains xanthan gum, then it does not contain iron trichloride.

The skincare composition according to the invention is advantageous primarily in that it is of sufficient viscosity to facilitate handling and dispensing of the composition, yet flows freely and is readily spreadable when applied to the intended site of application. In addition, the gel solubilises the salicylic acid effectively and is stable on storage.

According to another aspect of the invention, there is provided the use of a Copolymer of acryloyl dimethyl tauric acid or a salt thereof as a gelling agent and salicylic acid or a salt thereof in the manufacture of a skincare composition comprising a cosmetically acceptable hydroalcoholic gel, for the treatment of a disease or disorder of the skin, provided that if the composition contains xanthan gum, then it does not contain iron trichloride.

The use according to the latter aspect of the invention may have a therapeutic effect, in that it may be useful in the prophylaxis or remedial treatment of a disease or disorder of the skin, preferably acne. Alternatively, the use may be essentially cosmetic in nature, being effective to improve the appearance of the area of the skin to which the composition is applied.

According to a further aspect of the invention, there is provided the use of a copolymer of acryloyl dimethyl tauric acid or a salt thereof as a gelling agent in a cosmetically acceptable skincare composition comprising a hydroalcoholic gel comprising salicylic acid or a salt thereof.

The hydroalcoholic gel according to the invention is cosmetically acceptable to the consumer, namely, it has an acceptable appearance and odour. It may be applied to and left on the skin or it may be washed off, in both cases without leaving an undesirable residue on the skin or staining the skin or clothes. It may have a valuable cleansing effect on the skin. In addition, the viscosity remains substantially unchanged on storage, for example over a month or up to a year or even longer.

The gelling agent useful in the composition according to the invention is a copolymer of acryloyl dimethyl tauric acid (or a salt thereof), especially a copolymer of that monomer with another vinylic monomer.

Most preferably, the gelling agent is a copolymer of a salt of acryloyl dimethyl tauric acid with another vinylic monomer. The salt may be a salt of a Group I alkali metal, but is more preferably an ammonium salt.

Examples of suitable copolymer gelling agents are:
a) Ammonium acryloyl dimethyl taurate / vinyl pyrrolidone copolymer, ie a copolymer of ammonium acryloyl dimethyl taurate and vinyl pyrrolidone (1-vinyl-2-pyrrolidona). This material is available under the trade name Aristoflex AVC from Clariant GmbH, Functional Chemicals Division, D-65840 Sulzbach, Germany.
b) Ammonium acryloyl dimethyl taurate / Beheneth-25 methacrylate copolymer, ie a copolymer of ammonium acryloyl dimethyl taurate and Beheneth-25 methacrylate, the structure of which is

   CH₂=CH(CH₃)CO₂-(CH₂CH₂O)ₙCH₂(CH₂)₂₀CH₃

   in which n is approximately 25. This material is also available from Clariant GmbH under the trade name Aristoflex HMB.
c) Ammonium acryloyldimethyltaurate / vinyl formamide copolymer, ie a copolymer of ammonium acryloyl dimethyl taurate and vinyl formamide. Again, a suitable material is available from Clariant GmbH under the trade name Aristoflex AVC-1.

The composition most preferably comprises less than 10% w/w of the gelling agent, and more commonly less than 5% w/w. The amount of gelling agent will generally be greater than 0.1% w/w and more commonly greater than 0.5% w/w. The amount of gelling agent in the composition will preferably lie in the range 0.1 to 5% w/w, more preferably 0.5 to 5% w/w. Typically, the amount of gelling agent will be less than 3% w/w, eg about 1% w/w or about 2% w/w.

If desired, a proportion of the copolymer of acryloyl dimethyl tauric acid (or salt thereof) may be replaced by other gelling agents stable at low pH in the composition. Preferably, the acryloyl dimethyl tauric acid copolymer (or salt thereof) is the sole gelling agent in the composition. More preferably, ammonium acryloyl dimethyl tauric acid copolymer is the sole gelling agent in the composition.

Furthermore, thickening agents which increase the viscosity of the gel at or below pH 4.5 may also be employed. For example, water-soluble or hydrophilic materials are preferred, such as naturally occurring gums and cellulose or derivatives thereof. Particularly suitable thickeners are hydroxypropylmethyl cellulose or hydroxyethyl cellulose (available from Hercules under the trade name Natrosol), especially hydroxyethyl cellulose.

The amount of thickening agent in the composition may lie in the range 0 to 10% w/w. If the thickening agent is present, the composition may comprise from 0.1 to 5% w/w, more preferably 0.5 to 3% w/w. Generally, it is preferred to use a gelling agent in the absence of a thickening agent.

The composition according to the invention preferably has a viscosity of from about 50 mPa.s to about 20,000 mPa.s, more preferably from about 100 mPa.s to about 10,000 mPa.s. Viscosity may be measured using a Brookfield LVT viscometer equipped with a spindle 4 rotating at 12rpm after 2 minutes.

The gel according to the present invention is preferably free or substantially free of polyvalent ionic material, namely cations and anions, which may cause undesired cross-linking between the polymer chains which may affect the initial formation of the gel or change the viscosity of the gel on storage. By "substantially free", we mean less than 1% polyvalent ionic material, preferably less than 0.1% and especially less than 0.01% polyvalent ionic material.

On application to the skin, the gel is applied to a small area and is then rubbed over the skin. The high shear force of the rubbing action, the presence of salts on the skin and the interaction with increased area of the skin over which the gel is spread causes the gel substantially to break down or thin, namely the viscosity is reduced significantly. This effect causes it to be much easier to apply the composition to the skin without causing an undesirable residue. It also minimises waste of the product as very little of the composition is wasted on application of the gel, for example by being absorbed into cotton application pads. The thinning effect of the gel may be measured by standard rheology measurement, for example using a Physica MCR 301 (Anton Paar). The yield value also provides useful information about a gel. On rubbing the gel into the skin, the viscosity is generally reduced by a factor of at least 2, preferably by a factor of at least 4 and most preferably by a factor of at least 8. The initial viscosity of the gel may be reduced to less than 100 mPa.s, preferably less than 10mPa.s, on rubbing into the skin.

The composition according to the invention has the form of a hydroalcoholic gel. As such, the composition will generally comprise a major proportion of water. The amount of water in the composition will typically be in excess of 40% w/w, more commonly in excess of 50% w/w, and may be in excess of 75% w/w. The upper limit of water will depend on the amounts of other ingredients incorporated in the composition so that the water may form the remainder of the composition up to 100% of the composition. A typical maximum value is less than 90% by weight, for example 80% by weight or 85% by weight.

The composition according to the invention comprises an alcohol cosolvent which has a greater volatility than water, preferably a C₁₋₆ alcohol, more preferably a C₂₋₄ alkanol. The cosolvent is most preferably ethanol or isopropyl alcohol. Compositions comprising ethanol are particularly preferred.

The composition most preferably comprises in excess of 5% w/w of the cosolvent, and may comprise in excess of 10% w/w, in excess of 20% w/w, or in excess of 30% w/w of the cosolvent. The amount of cosolvent present in the composition preferably does not exceed 50% w/w. The amount of cosolvent thus preferably lies in the range 5% to 50% w/w, more preferably 10% to 50% w/w. In general, higher proportions of cosolvent may be required in compositions containing higher proportions of ingredients (eg topically active ingredients, as discussed below) that are of low solubility in water. Where such ingredients are absent, of their concentration is relatively low, the proportion of cosolvent may also be somewhat lower than in other embodiments, eg up to 20% w/w.

Overall, the concentration of solvent in the composition (ie water and cosolvent) is preferably in excess of 80% w/w, and may be in excess of 90% w/w. The total amount of solvent in the composition will generally be less than 99% w/w.

Salicylic acid is preferably incorporated into the composition according to the invention as the free acid. However, the pH of the composition may, and generally will, be such that the salicylic acid exists in the composition in dissociated form. As the composition may well contain cationic counterions, the salicylic acid may then be thought of as being present in salt form. Alternatively, the salicylic acid may be incorporated into the composition in salt form, eg as a salt with a Group I metal, such as sodium salicylate. As used herein, unless the context requires otherwise, any and all references to salicylic acid should be taken to encompass references to the acid and to dissociated forms and salts thereof. The salicylic acid may also be provided from naturally occurring sources, such as willow herb. Preferably, the salicylic acid is the sole active ingredient in a composition according to the present invention.

The concentration of salicylic acid in the composition according to the invention is preferably at least 0.1 % by weight, more preferably at least 0.5%. The concentration of salicylic acid is preferably less than 5%, more preferably less than 4%, and most preferably less than 3% by weight. The concentration of salicylic acid may therefore fall in the range 0.1 % to 5% by weight, more preferably 0.5% to 4%, and most preferably 0.5% to 3%. Particularly preferred concentrations of salicylic acid are 0.5%, 1%, 1.5% and 2% by weight.

The hydroalcoholic gel preferably has a pH of 5.5 or less, more preferably 4.5 or less, for example from 1 to 4.5, preferably from 2 to 4 and most preferably from 2.5 to 3.5, particularly about pH 3.

The composition according to the invention may also comprise one or more further topically active ingredients useful in skincare. Such active ingredients may include one or more of the following:
antimicrobial or antibacterial compounds, for example selected from the following:
triclosan, neomycin, clindamycin, polymyxin, bacitracin, benzoyl peroxide, hydrogen peroxide, tetracylines such as doxycycline or minocycline, sulfa drugs such as sulfacetamide, penicillins, cephalosporins such as cephalexin, and quinolones such as lomefloxacin, olfoxacin or trovafloxacin;
antiviral compounds, for example selected from acyclovir, tamvir, and penciclovir;
antifungal compounds, for example selected from the following: farnesol, clotrimazole, ketoconazole, econazole, fluconazole, calcium or zinc undecylenate, undecylenic acid, butenafine hydrochloride, ciclopirox olaimine, miconazole nitrate, nystatin, sulconazole, and terbinafine hydrochloride;
anti-inflammatory compounds, for example selected from the following: steroidal agents selected from hydrocortisone, fluocinolone acetonide, halcinonide, halobetasol propionate, clobetasol propionate, betamethasone dipropionate, betamethasone valerate, and triamcinolone acetanide, and non-steroidal anti-inflammatory agents selected from aspirin, ibuprofen, ketoprofen, naproxen, aloe vera gel, aloe vera, licorice extract, pilewort, Canadian willow root, zinc, and allantoin; anthelmintic compounds, for example metronidazole.

The composition may also comprise an antibacterial agent, particularly a peroxide antibacterial agent. A preferred peroxide antibacterial agent for inclusion in the composition is hydrogen peroxide. Alternatively, the composition may comprise a compound that, in use, is capable of generating hydrogen peroxide. An example of the latter class of compound is an adduct such as urea peroxide (carbamide peroxide).

In one preferred embodiment of the invention, the composition comprises both salicylic acid and hydrogen peroxide.

Where hydrogen peroxide is present in the composition according to the invention, the concentration of hydrogen peroxide is preferably at least 1 % by weight. The concentration of hydrogen peroxide is preferably less than 5%, more preferably less than 3%, and most preferably less than 2% by weight. The concentration of hydrogen peroxide may therefore fall within the range 1% to 5% by weight, more preferably 1% to 3%, and most preferably 1% to 2% by weight.

The composition is a dispersion and may be formulated in numerous forms, including a clear or transparent gel. However, the composition may often take the form of an emulsion, especially a cream gel. An emulsion may include an oil-in-water emulsion.

The oil phase of water-in-oil or oil-in-water emulsions may comprise for example:
a) hydrocarbon oils such as paraffin or mineral oils;
b) waxes such as beeswax or paraffin wax;
c) natural oils such as sunflower oil, apricot kernel oil, shea butter or jojoba oil;
d) silicone oils such as dimethicone, cyclomethicone or cetyldimethicone;
e) fatty acid esters such as isopropyl palmitate, isopropyl myristate, dioctylmaleate, glyceryl oleate and cetostearyl isononanoate;
f) fatty alcohols such as cetyl alcohol or stearyl alcohol and mixtures thereof (eg cetearyl alcohol);
g) polypropylene glycol or polyethylene glycol ethers, eg PPG-14 butyl ether; or
h) mixtures thereof, for example, the blend of waxes available commercially under the trade name Cutina (Henkel).

Emulsifiers used may be any emulsifiers known in the art for use in water-in-oil or oil-in-water emulsions. Known cosmetically acceptable emulsifiers include:
a) sesquioleates such as sorbitan sesquioleate, available commercially for example under the trade name Arlacel 83 (ICI), or polyglyceryl-2-sesquioleate;
b) ethoxylated esters of derivatives of natural oils such as the polyethoxylated ester of hydrogenated castor oil available commercially for example under the trade name Arlacel 989 (ICI);
c) silicone emulsifiers such as silicone polyols available commercially for example under the trade name ABIL WS08 (Th. Goldschmidt AG);
d) anionic emulsifiers such as fatty acid soaps e.g. potassium stearate and fatty acid sulphates e.g. sodium cetostearyl sulphate available commercially under the trade name Dehydag (Henkel);
e) ethoxylated fatty alcohols, for example the emulsifiers available commercially under the trade name Brij (ICI);
f) sorbitan esters, for example the emulsifiers available commercially under the trade name Span (ICl);
g) ethoxylated sorbitan esters, for example the emulsifiers available commercially under the trade name Tween (ICl);
h) ethoxylated fatty acid esters such as ethoxylated stearates, for example the emulsifiers available commercially under the trade name Myrj (ICl);
i) ethoxylated mono-, di-, and tri-glycerides, for example the emulsifiers available commercially under the trade name Labrafil (Alfa Chem.);
j) non-ionic self-emulsifying waxes, for example the wax available commercially under the trade name Polawax (Croda);
k) ethoxylated fatty acids, for example, the emulsifiers available commercially under the trade name Tefose (Alfa Chem.);
l) methylglucose esters such as polyglycerol-3 methyl glucose distearate available commercially under the name Tegocare 450 (Degussa Goldschmidt); or
m) mixtures thereof.

The composition according to the invention may additionally comprise other components which will be well known to those skilled in the art. These include, for example:
a) Surfactants - Surfactants may be used in compositions according to the invention as solubilisers, or as cleansing agents or foam boosters. Many different classes of surfactant may be suitable for inclusion in the composition according to the invention, and these will be readily apparent to those skilled in the art. Examples of suitable surfactants include polyethylene glycol ethers of alcohols such as isocetyl alcohol (eg lsoceteth-20), isostearyl alcohol (eg Isosteareth-20), cetyl alcohol (eg Ceteth-20), oleyl alcohol (eg Oleth-20) and cetearyl alcohol (eg Ceteareth-20). A particularly preferred surfactant for use in the invention is Isoceteth-20.
b) Emollients - ingredients that help to maintain the soft, smooth and pliable appearance of skin. Such ingredients may function by their ability to remain on the surface of the skin or in the stratum corneum, and to act as lubricants, reducing or preventing flaking of the skin and improving the skin's appearance. Examples of emollients are isopropyl myristate, triglycerides of fatty acids eg lauric triglyceride or capric/caprylic triglyceride, such as the tri-glyceride available commercially under the trade name Miglyol 810 (Huls UK), and the polypropylene glycol ether of stearyl alcohol known as PPF-15 Stearyl Ether. Particularly preferred emollients are polysiloxane compounds, in particular those known as cyclomethicone, ie cyclic dimethyl polysiloxane compounds that conform to the formula:

   -(Si(CH₃)₂)ₙ-

   in which n has a value between 3 and 7.
c) Humectants or Moisturisers - ingredients intended to increase the water content of the top layers of the skin. Examples of such ingredients are glycerin, 1,3-butylene glycol and propylene glycol.
d) Preservatives - ingredients which prevent or retard microbial growth and thus protect the composition from spoilage. Examples of preservatives include such as propylparaben, bronopol, sodium dehydroacetate, polyhexamethylenebiguanide hydrochloride, isothiazolone and diazolidinylurea.
e) Chelating agents or sequestering agents (sequestrants) - ingredients that have the ability to complex with and inactivate metallic ions in order to prevent their adverse effects on the stability or appearance of the composition. Examples of chelating agents are ethylenediamine tetraacetic acid and its salts, notably the dipotassium and especially the disodium or tetrasodium salt.
f) pH adjusters - Ingredients used to control the pH of the composition. Examples of pH adjusters are inorganic salts such as sodium hydroxide, and organic bases such as triethanolamine. The pH of the composition is preferably in the range of pH 3-6, and more preferably in the range pH 3-5.
g) mattifying agents - Ingredients used to reduce shine and to impart a matt appearance to the skin to which the composition is applied. Such agents commonly comprise particulate, oil-absorbent polymers. A preferred example of a suitable mattifying agent is lauryl methacrylate/glycol dimethacrylate crosspolymer, which is available under the tradename POLYTRAP Q5-6603.
h) oil control agents- ingredients used to control the rate of sebum production by the skin, such as sebum regulators which regulate the number of active glands or oil absorbing agents which remove excess oil form the skin. A preferred oil control agent is hydrolysed milk protein.
i) Perfumes and colourings.

The composition according to the invention may be applied and left on the skin to have the desired therapeutic effect or it may be applied and then rinsed off, for example with water. The composition may be applied with the aid of a fibrous material, for example a pad or a wipe.

An article comprising a fibrous substrate, for example a material in the form of a pad or a wipe, may be impregnated with a cosmetically acceptable skincare composition in the form of a hydroalcoholic gel dispersion, the composition comprising salicylic acid or a salt thereof and a gelling agent in the form of a copolymer of acryloyl dimethyl tauric acid or a salt thereof.

Said fibrous substrate may be impregnated with the skincare composition in an amount in the range from 10 to 30% by weight, preferably from 15 to 25% by weight and most preferably from 18 to 22% by weight of the fibrous substrate. Suitable fibrous substrates comprise materials which include natural or synthetic fibres or a mixture thereof, for example cellulose and/or cotton fibres. The fibrous substrate may be impregnated with the composition as a wet wipe which is arranged for immediate use to apply the skincare composition of the present invention to the skin of the user. Alternatively, the fibrous substrate may be impregnated with the skincare composition and dried to form a dry wipe which requires to be wetted, for example with water, before it can be used.

The invention will now be described in greater detail, by way of illustration only, with reference to the following Examples.

### Example 1

### Gel Lotion

| Ingredients | % w/w |
|---|---|
| Aqua | to 100% |
| Alcohol denat. | 35% |
| lsoceteth-20 | 3.0% |
| Salicylic acid | 2.0% |
| Hydrogen peroxide (35%) | 4.286% |
| Ammonium acryloyldimethyltaurate/ vinyl pyrrolidone copolymer | 2.0% |
| Sodium hydroxide (30%) | 0.4% |
| Parfum | 0.1 % |
| Disodium EDTA | 0.005% |

### Method

The salicylic acid was dissolved in the alcohol. When fully dispersed, water and the disodium EDTA were added. The ammoniumacryloyldimethyltaurate / vinyl pyrrolidine copolymer powder was sheared into the alcoholic mixture until lump free. The isoceteth-20, parfum and hydrogen peroxide were then stirred in, followed by adjustment of the pH to pH 3 with sodium hydroxide to form a composition according to the present invention.

### Example 2

### Gel Lotion

| ingredients | % w/w |
|---|---|
| Aqua | to 100% |
| Alcohol denat. | 15% |
| Isoceteth-20 | 1.0% |
| Salicylic acid | 0.5% |
| Ammonium acryloyldimethyltaurate/ vinyl pyrrolidone copolymer | 1.0% |
| Sodium hydroxide (30%) | 0.202% |
| Tetrasodium EDTA | 0.005% |

### Method

The salicylic acid was dissolved in the alcohol. When fully dispersed, the water and the tetrasodium EDTA were added. The ammonium acryloyldimethyltaurate / vinyl pyrrolidine copolymer powder was sheared into the alcoholic mixture until lump free. The isoceteth-20 and parfum were stirred into the thickened dispersion, and then the pH adjusted to pH 3 with sodium hydroxide to form a composition according to the present invention.

### Example 3

### Gel Lotion

| Ingredients | % w/w |
|---|---|
| Aqua | to 100% |
| Alcohol denat. | 15% |
| Isoceteth-20 | 1.0% |
| Salicylic acid | 0.5% |
| Ammonium acryloyldimethyltaurate/ vinyl pyrrolidone copolymer | 1.0% |
| Sodium hydroxide (30%) | 0.202% |
| Cyclomethicone | 3.0% |
| Lauryl methacrylate/glycol dimethacrylate crosspolymer | 0.5% |
| Tetrasodium EDTA | 0.005% |

### Method

The salicylic acid was dissolved in the alcohol. When fully dispersed, the water and the tetrasodium EDTA were added. The ammoniumacryloyldimethyltaurate / vinyl pyrrolidine copolymer powder was sheared into the alcoholic mixture until lump free. With continued shearing, the cyclomethicone and lauryl methacrylate / glycol dimethacrylate crosspolymer were added, and further sheared until a homogeneous mixture was formed. The isoceteth-20 and parfum were stirred into the thickened dispersion, and then the pH adjusted to pH 3 with sodium hydroxide to form a composition according to the present invention.

### Example 4

### Gel Lotion

| Ingredients | % w/w |
|---|---|
| Aqua | to 100% |
| Alcohol (99.9%) + t-butylalcohol (0.1 %) | 11.5% |
| Glycerin | 0.5% |
| lsoceteth-20 | 1.0% |
| Salicylic acid | 0.5% |
| Hydrogen peroxide (35%) | 4.28571% |
| Ammonium acryloyldimethyltaurate/ vinyl pyrrolidone copolymer | 1.5% |
| Hydrolyzed Milk Peptide | 0.2% |
| Sodium hydroxide (30%) | 0.4% |
| Parfum | 0.2% |
| Disodium EDTA | 0.005% |
| Colorant Cl 42090 (Blue No 1 FD&C) | 0.0003% |

### Method

The salicylic acid was mixed into the alcohol/t-butylalcohol. When the salicylic acid was fully dissolved, the water, glycerin and disodium EDTA were mixed in. The ammonium acryloyldimethyltaurate / vinyl pyrrolidone copolymer was then added with continuous homogenisation, followed by addition of the isoceteth-20, hydrogen peroxide, hydrolysed milk peptide and parfum in the water. The pH was adjusted to pH 3 with sodium hydroxide (30%) to form a composition according to the present invention.

### Example 5

### Gel Lotion

| Ingredients | % w/w |
|---|---|
| Aqua | to 100% |
| Alcohol (denat) | 15.0% |
| Isoceteth-20 | 1.0% |
| Ammonium acryloyldimethyltaurate/ vinyl pyrrolidone copolymer | 1.5% |
| Salicylic Acid | 0.5% |
| Sodium Citrate | 0.1 % |
| Parfum | 0.1% |
| Tetrasodium EDTA | 0.05% |

### Method

The salicylic acid was dissolved in the alcohol. When fully dispersed, the water and the sodium citrate were added, followed by shearing the ammonium acryloyldimethyltaurate / vinyl pyrrolidine copolymer powder into the alcoholic mixture until lump free. The isoceteth-20 and parfum were stirred into the thickened dispersion to form a composition according to the present invention.

### Example 6

### Gel Lotion

| Ingredients | % w/w |
|---|---|
| Aqua | to 100% |
| Alcohol (denat) | 15.0% |
| lsoceteth-20 | 1.0% |
| Ammonium acryloyldimethyltaurate/ vinyl pyrrolidone copolymer | 1.0% |
| Salicylic Acid | 0.5% |
| Sodium Hydroxide Solution (30%) | 0.2 % |
| Hydroxyethylcellulose | 0.2% |
| Tetrasodium EDTA | 0.005% |

### Method

The hydroxyethylcellulose and tetrasodium EDTA were sheared into the water until lump free and then the dispersion heated to 70°C to thicken. The dispersion was cooled to 35°C and the salicylic acid predissolved in the alcohol was added. The ammonium acryloyldimethyltaurate / vinyl pyrrolidine copolymer powder was sheared into the alcoholic mixture until lump free. The isoceteth-20 and parfum were both stirred into the thickened dispersion, and then the pH adjusted to pH 3 with sodium hydroxide to form a composition according to the present invention.

### Example 7

### Cream Gel

| Ingredients | % w/w |
|---|---|
| Aqua | to 100% |
| Ammonium acryloyldimethyltaurate/ Vinyl pyrrolidone copolymer | 0.4% |
| Ethylhexyl stearate | 6.0% |
| Glycerin | 5.0% |
| Cyclomethicone | 5.0% |
| Salicylic acid | 1.0% |
| Propylene glycol | 5.0% |
| Steareth-2 | 1.0% |
| Steareth-21 | 2.0% |
| Cetyl Alcohol | 2.5% |
| Propyl Hydroxybenzoate | 0.15% |
| Phenoxetol NIPA | 0.6% |
| Centella asiatica | 1.0% |
| Mimosa extract | 5.0% |
| Tetrasodium EDTA | 0.05 |
| Methyl Hydroxybenzoate | 0.25 |

### Method

The tetrasodium EDTA was mixed into 60% of the water until dissolved. The methyl hydroxybenzoate premixed into the glycerin was then added. The ammonium acryloyldimethyltaurate/ Vinyl pyrrolidone copolymer was added and homogenised in the resultant solution until a lump free dispersion was obtained. In a separate beaker the Steareth-2, Steareth-21, Ethylhexyl stearate, Cetyl Alcohol, Cyclomethicone and Propyl Hydroxybenzoate were heated until a temperature of 70 - 75°C is reached. This mixture was added to the copolymer dispersion mixture with homogenisation. Homogenising was continued for a further 5 minutes. The resultant dispersion was cooled down to 35°C and then a predispersion of Centella asiatica and mimosa extract in propylene glycol mixed in to form a composition according to the present invention.

## Claims

1. A cosmetically acceptable skincare composition in the form of a hydroalcoholic gel dispersion, the composition comprising salicylic acid or a salt thereof and a gelling agent in the form of a copolymer of acryloyl dimethyl tauric acid or a salt thereof, provided that if the composition contains xanthan gum, then it does not contain iron trichloride.

2. A composition as claimed in Claim 1, wherein the copolymer of acryloyl dimethyl tauric acid, or a salt thereof, is a copolymer of that monomer with another vinylic monomer.

3. A composition as claimed in Claim 1 or Claim 2, wherein the gelling agent is a copolymer of a salt of acryloyl dimethyl tauric acid with another vinylic monomer.

4. A composition as claimed in Claim 3, wherein the salt is an ammonium salt.

5. A composition as claimed in any one of Claims 1 to 3, wherein the gelling agent is selected from the group consisting of:
ammonium acryloyl dimethyl taurate / vinyl pyrrolidone copolymer; ammonium acryloyl dimethyl taurate / Beheneth-25 methacrylate copolymer; and
ammonium acryloyldimethyltaurate / vinyl formamide copolymer.

6. A composition as claimed in Claim 5, wherein the gelling agent is ammonium acryioyi dimethyl taurate / vinyl pyrrolidone copolymer.

7. A composition as claimed in any preceding claim, wherein the composition comprises less than 10% w/w of the gelling agent.

8. A composition as claimed in Claim 7, wherein the composition comprises less than 5% w/w of the gelling agent.

9. A composition as claimed in any preceding claim, wherein the composition comprises more than 0.1 % w/w of the gelling agent.

10. A composition as claimed in Claim 9, wherein the composition comprises more than 0.5% w/w of the gelling agent.

11. A composition as claimed in any preceding claim, wherein the composition comprises an amount of gelling agent in the range 0.1 to 5% w/w.

12. A composition as claimed in any preceding claim, wherein the amount of water in the composition is in excess of 40% w/w.

13. A composition as claimed in Claim 12, wherein the amount of water in the composition is in excess of 50% w/w.

14. A composition as claimed in Claim 13, wherein the amount of water in the composition is in excess of 75% w/w.

15. A composition as claimed in any preceding claim, wherein the hydroalcoholic gel comprises a C₁₋₆ alcohol as cosolvent.

16. A composition as claimed in Claim 15, wherein the cosolvent is a ethanol and/or isopropyl alcohol.

17. A composition as claimed in Claim 16, wherein the cosolvent is ethanol.

18. A composition as claimed in any one of Claims 15 to 17, which comprises in excess of 5% w/w of the cosolvent.

19. A composition as claimed in Claim 18, which comprises in excess of 10% w/w of the cosolvent.

20. A composition as claimed in Claim 19, which comprises in excess of 20% w/w of the cosolvent.

21. A composition as claimed in Claim 20, which comprises in excess of 30% w/w of the cosolvent.

22. A composition as claimed in any one of Claims 15 to 21, wherein the amount of cosolvent present in the composition does not exceed 50% w/w.

23. A composition as claimed in any preceding claim, wherein the active ingredient is salicylic acid.

24. A composition as claimed in Claim 23, wherein the concentration of salicylic acid in the composition is at least 0.1 % by weight.

25. A composition as claimed in Claim 24, wherein the concentration of salicylic acid in the composition is at least 0.5% by weight.

26. A composition as claimed in any one of Claims 23 to 25, wherein the concentration of salicylic acid is less than 5% by weight.

27. A composition as claimed in Claim 26, wherein the concentration of salicylic acid is less than 3% by weight.

28. A composition as claimed in any preceding claim, which comprises one or more further topically active ingredients useful in skincare.

29. A composition as claimed in Claim 28, wherein said one or more topically active ingredients are selected from the group consisting of:
antimicrobial or antibacterial compounds selected from the following:
triclosan, neomycin, clindamycin, polymyxin, bacitracin, benzoyl peroxide, hydrogen peroxide, tetracylines such as doxycycline or minocycline, sulfa drugs such as sulfacetamide, penicillins, cephalosporins such as cephalexin, and quinolones such as lomefloxacin, olfoxacin or trovafloxacin;
antiviral compounds, selected from acyclovir, tamvir, and penciclovir; antifungal compounds, selected from the following: farnesol, clotrimazole, ketoconazole, econazole, fluconazole, calcium or zinc undecylenate, undecylenic acid, butenafine hydrochloride, ciclopirox olaimine, miconazole nitrate, nystatin, sulconazole, and terbinafine hydrochloride;
anti-inflammatory compounds, selected from the following: steroidal agents selected from hydrocortisone, fluocinolone acetonide, halcinonide, halobetasol propionate, clobetasol propionate, betamethasone dipropionate, betamethasone valerate, and triamcinolone acetonide, and non-steroidal anti-inflammatory agents selected from aspirin, ibuprofen, ketoprofen, naproxen, aloe vera gel, aloe, vera, licorice extract, pilewort, Canadian willow root, zinc, and allantoin; and metronidazole.

30. A composition as claimed in any one of Claims 23 to 29, wherein the composition also comprises an antibacterial agent.

31. A composition as claimed in Claim 30, wherein the antibacterial agent is a peroxide antibacterial agent.

32. A composition as claimed in Claim 31, wherein the peroxide antibacterial agent is hydrogen peroxide or the composition comprises a compound that, in use, is capable of generating hydrogen peroxide.

33. A composition as claimed in Claim 32, wherein the concentration of hydrogen peroxide is at least 1% by weight.

34. A composition as claimed in Claim 32 or Claim 33, wherein the concentration of hydrogen peroxide is less than 5% by weight.

35. A composition as claimed in Claim 34, wherein the concentration of hydrogen peroxide is less than 2% by weight.

36. The use of a copolymer of acryloyl dimethyl tauric acid or a salt thereof as a gelling agent in a cosmetically acceptable skincare composition comprising a hydroalcoholic gel comprising salicylic acid or a salt thereof, provided that if the composition contains xanthan gum, then it does not contain iron trichloride.

37. The use of a copolymer of acryloyl dimethyl tauric acid or a salt thereof as a gelling agent and salicylic acid or a salt thereof in the manufacture of a skincare composition comprising a cosmetically acceptable hydroalcoholic gel for the treatment of a disease or disorder of the skin, provided that if the composition contains xanthan gum, then it does not contain iron trichloride.

38. The use as claimed in Claim 36 to or Claim 37, wherein the composition is a composition as claimed in any one of Claims 2 to 35.

## Patentansprüche

1. Kosmetisch verträgliche Hautpflegezusammensetzung in Form einer wässrig-alkoholischen Geldispersion, wobei die Zusammensetzung Salicylsäure oder ein Salz davon und einen Gelbildner in Form eines Copolymers von Acryloyldimethyltaurinsäure oder eines Salzes davon umfasst, mit der Maßgabe, dass, wenn die Zusammensetzung Xanthangummi enthält, sie dann nicht Eisentrichlorid enthält.

2. Zusammensetzung nach Anspruch 1, wobei das Copolymer von Acryloyldimethyltaurinsäure oder eines Salzes davon ein Copolymer von diesem Monomer mit einem anderen Vinylmonomer ist.

3. Zusammensetzung nach Anspruch 1 oder Anspruch 2, wobei der Gelbildner ein Copolymer eines Salzes von Acryloyldimethyltaurinsäure mit einem anderen Vinylmonomer ist.

4. Zusammensetzung nach Anspruch 3, wobei das Salz ein Ammoniumsalz ist.

5. Zusammensetzung nach einem der Ansprüche 1 bis 3, wobei der Gelbildner ausgewählt ist aus der Gruppe, bestehend aus Ammoniumacryloyldimethyltaurat/Vinylpyrrolidon-Copolymer; Ammoniumacryloyldimethyltaurat/Beheneth-25-methacrylat-Copolymer; und Ammoniumacryloyldimethyltaurat/Vinylformamid-Copolymer.

6. Zusammensetzung nach Anspruch 5, wobei der Gelbildner Ammoniumacryloyldimethyltaurat/Vinylpyrrolidon-Copolymer ist.

7. Zusammensetzung nach einem der vorangehenden Ansprüche, wobei die Zusammensetzung weniger als 10 Gew.-% des Gelbildners umfasst.

8. Zusammensetzung nach Anspruch 7, wobei die Zusammensetzung weniger als 5 Gew.-% des Gelbildners umfasst.

9. Zusammensetzung nach einem der vorangehenden Ansprüche, wobei die Zusammensetzung mehr als 0,1 Gew.-% des Gelbildners umfasst.

10. Zusammensetzung nach Anspruch 9, wobei die Zusammensetzung mehr als 0,5 Gew.-% des Gelbildners umfasst.

11. Zusammensetzung nach einem der vorangehenden Ansprüche, wobei die Zusammensetzung eine Gelbildnermenge im Bereich von 0,1 bis 5 Gew.- % umfasst.

12. Zusammensetzung nach einem der vorangehenden Ansprüche, wobei die Wassermenge in der Zusammensetzung über 40 Gew.-% liegt.

13. Zusammensetzung nach Anspruch 12, wobei die Wassermenge in der Zusammensetzung über 50 Gew.-% liegt.

14. Zusammensetzung nach Anspruch 13, wobei die Wassermenge in der Zusammensetzung über 75 Gew.-% liegt.

15. Zusammensetzung nach einem der vorangehenden Ansprüche, wobei das wässrig-alkoholische Gel einen C₁₋₆-Alkohol als weiteres Lösungsmittel umfasst.

16. Zusammensetzung nach Anspruch 15, wobei das weitere Lösungsmittel Ethanol und/oder Isopropylalkohol ist.

17. Zusammensetzung nach Anspruch 16, wobei das weitere Lösungsmittel Ethanol ist.

18. Zusammensetzung nach einem der Ansprüche 15 bis 17, die über 5 Gew.- % des weiteren Lösungsmittels umfasst.

19. Zusammensetzung nach Anspruch 18, die über 10 Gew.-% des weiteren Lösungsmittels umfasst.

20. Zusammensetzung nach Anspruch 19, die über 20 Gew.-% des weiteren Lösungsmittels umfasst.

21. Zusammensetzung nach Anspruch 20, die über 30 Gew.-% des weiteren Lösungsmittels umfasst.

22. Zusammensetzung nach einem der Ansprüche 15 bis 21, wobei die in der Zusammensetzung vorliegende Menge des weiteren Lösungsmittels 50 Gew.-% nicht übersteigt.

23. Zusammensetzung nach einem der vorangehenden Ansprüche, wobei der Wirkstoff Salicylsäure ist.

24. Zusammensetzung nach Anspruch 23, wobei die Konzentration an Salicylsäure in der Zusammensetzung mindestens 0,1 Gew.-% beträgt.

25. Zusammensetzung nach Anspruch 24, wobei die Konzentration an Salicylsäure in der Zusammensetzung mindestens 0,5 Gew.-% beträgt.

26. Zusammensetzung nach einem der Ansprüche 23 bis 25, wobei die Konzentration an Salicylsäure weniger als 5 Gew.-% beträgt.

27. Zusammensetzung nach Anspruch 26, wobei die Konzentration an Salicylsäure weniger als 3 Gew.-% beträgt.

28. Zusammensetzung nach einem der vorangehenden Ansprüche, die einen oder mehrere weitere topisch wirksame Inhaltstoffe umfasst, die bei der Hautpflege nützlich sind.

29. Zusammensetzung nach Anspruch 28, wobei der eine oder die mehreren topisch wirksamen Inhaltsstoffe ausgewählt sind aus der Gruppe, bestehend aus: antimikrobiellen und antibakteriellen Verbindungen, ausgewählt aus Folgendem: Triclosan, Neomycin, Clindamycin, Polymyxin, Bacitracin, Benzoylperoxid, Wasserstoffperoxid, Tetracyclinen wie Doxycyclin oder Minocyclin, Sulfaarzneistoffen wie Sulfacetamid, Penicillinen, Cephalosporinen wie Cephalexin und Chinolonen wie Lomefloxacin, Olfoxacin oder Trovafloxacin; antiviralen Verbindungen ausgewählt aus Acyclovir, Tamvir und Penciclovir; antimykotische Verbindungen, ausgewählt aus Folgendem: Farnesol, Clotrimazol, Ketoconazol, Econazol, Fluconazol, Calcium- oder Zinkundecylenat, Undecylensäure, Butenafinhydrochlorid, Ciclopiroxolaimin, Miconazolnitrat, Nystatin, Sulconazol und Terbinafinhydrochlorid; entzündungshemmenden Verbindungen, ausgewählt aus Folgendem: steroiden Mitteln, ausgewählt aus Hydrocortison, Fluocinolonacetonid, Halcinonid, Halobetasolpropionat, Clobetasolpropionat, Betamethasondipropionat, Betamethasonvalerat und Triamcinolonacetonid, und nicht-steroiden entzündungshemmenden Mitteln, ausgewählt aus Aspirin, Ibuprofen, Ketoprofen, Naproxen, Aloevera-Gel, Aloe vera, Süßholzextrakt, Scharbockskraut, Kanadischer Weidenwurzel, Zink und Allantoin; und Metronizadol.

30. Zusammensetzung nach einem der Ansprüche 23 bis 29, wobei die Zusammensetzung auch ein antibakterielles Mittel umfasst.

31. Zusammensetzung nach Anspruch 30, wobei das antibakterielle Mittel ein antibakterielles Peroxidmittel ist.

32. Zusammensetzung nach Anspruch31, wobei das antibakterielle Peroxidmittel Wasserstoffperoxid ist oder die Zusammensetzung eine verbindung umfasst, die bei Verwendung in der Lage ist, Wasserstoffperoxid zu erzeugen.

33. Zusammensetzung nach Anspruch 32, wobei die Konzentration von Wasserstoffperoxid mindestens 1 Gew.-% beträgt.

34. Zusammensetzung nach Anspruch 32 oder Anspruch 33, wobei die Konzentration von Wasserstoffperoxid weniger als 5 Gew.-% beträgt.

35. Zusammensetzung nach Anspruch 34, wobei die Konzentration von Wasserstoffperoxid weniger als 2 Gew.-% beträgt.

36. Verwendung eines Copolymers von Acrylolyldimethyltaurinsäure oder eines Salzes davon als Gelbildner in einer kosmetisch verträglichen Hautpflegezusammensetzung, umfassend ein wässrig-alkoholisches Gel, umfassend Salicylsäure oder ein Salz davon, mit der Maßgabe, dass, wenn die Zusammensetzung Xanthangummi enthält, sie dann nicht Eisentrichlorid enthält.

37. Verwendung eines Copolymers von Acrylolyldimethyltaurinsäure oder eines Salzes davon als Gelbildner und Salicylsäure oder ein Salz davon bei der Herstellung einer Hautpflegezusammensetzung, umfassend ein kosmetisch verträgliches wässrig-alkoholisches Gel, zur Behandlung einer Erkrankung oder Störung der Haut, mit der Maßgabe, dass, wenn die Zusammensetzung Xanthangummi enthält, sie dann nicht Eisentrichlorid enthält.

38. Verwendung nach Anspruch 36 oder 37, wobei die Zusammensetzung eine Zusammensetzung nach einem der Ansprüche 2 bis 35 ist.

## Revendications

1. Composition pour les soins cutanés, acceptable du point de vue cosmétique, sous forme d'une dispersion dans un gel hydroalcoolique, la composition comprenant de l'acide salicylique ou un de ses sels et un agent gélifiant sous forme d'un copolymère d'acide acryloyldiméthyltaurique ou d'un de ses sels, sous réserve que, si la composition contient de la gomme xanthane, alors elle ne contient pas de trichlorure de fer.

2. Composition suivant la revendication 1, dans laquelle le copolymère d'acide acryloyldiméthyltaurique, ou d'un de ses sels, est un copolymère de ce monomère avec un autre monomère vinylique.

3. Composition suivant la revendication 1 ou la revendication 2, dans laquelle l'agent gélifiant est un copolymère d'un sel d'acide acryloyldiméthyltaurique avec un autre monomère vinylique.

4. Composition suivant la revendication 3, dans laquelle le sel est un sel d'ammonium.

5. Composition suivant l'une quelconque des revendications 1 à 3, dans lequel l'agent gélifiant est choisi dans le groupe consistant en :
un copolymère acryloyldiméthyltaurate d'ammonium/vinyl-pyrrolidone ;
un copolymère acryloyldiméthyltaurate d'ammonium/méthacrylate de béhéneth-25 ;
et
un copolymère acryloyldiméthyltaurate d'ammonium/vinyl-formamide.

6. Composition suivant la revendication 5, dans laquelle l'agent gélifiant est un copolymère acryloyldiméthyltaurate d'ammonium/vinylpyrrolidone.

7. Composition suivant l'une quelconque des revendications précédentes, qui comprend moins de 10 % en poids/poids de l'agent gélifiant.

8. Composition suivant la revendication 7, qui comprend moins de 5 % en poids/poids de l'agent gélifiant.

9. Composition suivant l'une quelconque des revendications précédentes, qui comprend plus de 0,1 % en poids/poids de l'agent gélifiant.

10. Composition suivant la revendication 9, qui comprend plus de 0,5 % en poids/poids de l'agent gélifiant.

11. Composition suivant l'une quelconque des revendications précédentes, qui comprend une quantité d'agent gélifiant comprise dans l'intervalle de 0,1 à 5 % en poids/poids.

12. Composition suivant l'une quelconque des revendications précédentes, dans laquelle la quantité d'eau dans la composition est supérieure à 40 % en poids/poids.

13. Composition suivant la revendication 12, dans laquelle la quantité d'eau dans la composition est supérieure à 50 % en poids/poids.

14. Composition suivant la revendication 13, dans laquelle la quantité d'eau dans la composition est supérieure à 75 % en poids/poids.

15. Composition suivant l'une quelconque des revendications précédentes, dans laquelle le gel hydroalcoolique comprend un alcool en C₁ à C₆ comme cosolvant.

16. Composition suivant la revendication 15, dans laquelle le cosolvant est un éthanol et/ou alcool isopropylique.

17. Composition suivant la revendication 16, dans laquelle le cosolvant est l'éthanol.

18. Composition suivant l'une quelconque des revendications 15 à 17, qui comprend plus de 5 % en poids/poids du cosolvant.

19. Composition suivant la revendication 18, qui comprend plus de 10 % en poids/poids du cosolvant.

20. Composition suivant la revendication 19, qui comprend plus de 20 % en poids/poids du cosolvant.

21. Composition suivant la revendication 20, qui comprend plus de 30 % en poids/poids du cosolvant.

22. Composition suivant l'une quelconque des revendications 15 à 21, dans laquelle la quantité de cosolvant présente dans la composition ne dépasse pas 50 % en poids/poids.

23. Composition suivant l'une quelconque des revendications précédentes, dans laquelle l'ingrédient actif est l'acide salicylique.

24. Composition suivant la revendication 23, dans laquelle la concentration d'acide salicylique dans la composition est égale à au moins 0,1 % en poids.

25. Composition suivant la revendication 24, dans laquelle la concentration d'acide salicylique dans la composition est égale à au moins 0,5 % en poids.

26. Composition suivant l'une quelconque des revendications 23 à 25, dans laquelle la concentration d'acide salicylique est inférieure à 5 % en poids.

27. Composition suivant la revendication 26, dans laquelle la concentration d'acide salicylique est inférieure à 3 % en poids.

28. Composition suivant l'une quelconque des revendications précédentes, qui comprend un ou plusieurs ingrédients topiquement actifs supplémentaires utiles dans les soins cutanés.

29. Composition suivant la revendication 28, dans laquelle ledit ou lesdits ingrédients topiquement actifs sont choisis dans le groupe consistant en :
des composés antimicrobiens ou antibactériens choisis parmi les suivants : triclosan, néomycine, clindamycine, polymyxine, bacitracine, peroxyde de benzoyle, peroxyde d'hydrogène, tétracyclines telles que doxycycline ou minocycline, sulfa-médicaments tels que sulfacétamide, pénicillines, céphalosporines telles que céphalexine, et quinolones telles que loméfloxacine, ofloxacine ou trovafloxacine ; composés antiviraux choisis entre acyclovir, tamvir et penciclovir ; composés antifongiques choisis parmi les suivants : farnésol, clotrimazole, kétoconazole, éconazole, fluconazole, undécylénate de calcium ou de zinc, acide undécylénique, chlorhydrate de buténafine, ciclopirox-olaimine, nitrate de miconazole, nystatine, sulconazole et chlorhydrate de terbinafine ; composés anti-inflammatoires choisis parmi les suivants :
agents stéroïdiens choisis entre hydrocortisone, fluocinolone-acétonide, halcinonide, propionate d'halobétasol, propionate de clobétasol, dipropionate de bétaméthasone, valérate de bétaméthasone et triamcinolone-acétonide et
agents anti-inflammatoires non stéroïdiens choisis entre aspirine, ibuprofène, kétoprofène, naproxène, gel d'Aloe vera, Aloe vera, extrait de réglisse, ficaire, racine de saule du Canada, zinc et allantoïne ; et métronidazole.

30. Composition suivant l'une quelconque des revendications 23 à 29, qui comprend également un agent antibactérien.

31. Composition suivant la revendication 30, dans laquelle l'agent antibactérien est un agent antibactérien du type peroxyde.

32. Composition suivant la revendication 31, dans laquelle l'agent antibactérien du type peroxyde est le peroxyde d'hydrogène, ou bien la composition comprend un composé qui, lors de l'utilisation, est capable d'engendrer du peroxyde d'hydrogène.

33. Composition suivant la revendication 32, dans laquelle la concentration de peroxyde d'hydrogène est égale à au moins 1 % en poids.

34. Composition suivant la revendication 32 ou la revendication 33, dans laquelle la concentration de peroxyde d'hydrogène est inférieure à 5 % en poids.

35. Composition suivant la revendication 34, dans laquelle la concentration de peroxyde d'hydrogène est inférieure à 2 % en poids.

36. Utilisation d'un copolymère d'acide acryloyldiméthyltaurique ou d'un de ses sels comme agent gélifiant dans une composition pour les soins cutanés acceptable du point de vue cosmétique comprenant un gel hydroalcoolique comprenant de l'acide salicylique ou un de ses sels, sous réserve que, si la composition contient de la gomme xanthane, alors elle ne contient pas de trichlorure de fer.

37. Utilisation d'un copolymère d'acide acryloyldiméthyltaurique ou d'un de ses sels comme agent gélifiant et d'acide salicylique ou d'un de ses sels dans la production d'une composition pour les soins cutanés comprenant un gel hydroalcoolique acceptable du point de vue cosmétique pour le traitement d'une maladie ou d'un trouble de la peau, ou sous réserve que, si la composition contient de la gomme xanthane, alors elle ne contient pas de trichlorure de fer.

38. Utilisation suivant la revendication 36 ou la revendication 37, dans laquelle la composition est une composition suivant l'une quelconque des revendications 2 à 35.
